# EUROPEAN PATENT APPLICATION

(11) **EP 4 736 907 A1**
(43) Date of publication of application: **06.05.2026**
(21) Application number: 24306853.3
(22) Date of filing: 04.11.2024
(51) Int. Cl.: A61M 5/142, A61M 5/145, A61M 5/148, A61M 5/155, A61M 5/168

(54) **A DRUG DELIVERY SYSTEM**

(71) Applicant: Becton Dickinson France, 38800 Le Pont-de-Claix (FR)
(72) Inventor: DELOBELLE, Vincent, 38420 DOMENE (FR); KALLEL, Hanen, 38000 GRENOBLE (FR)
(74) Representative: Germain Maureau

(57) **Abstract**

The drug delivery system (1), comprises:
- a first bag (21) containing a drug in liquid form;
- a second bag (22) configured to be inflated to compress the first bag for expelling the drug;
- a gas supply system (30) comprising a gas container (31) and a gas connector (40) for fluidly connecting the gas container to the second bag inlet (25), to inflate the second bag;
- a dispensing system (50) comprising an injection device (51) and a liquid connector (55) for fluidly connecting the first bag outlet (23) to the injection device inlet (52), wherein:
- in the inactive state of the dispensing system, the liquid connector blocks the fluid communication and the injection device is in a retracted position,
- in the active state of the dispensing system, the liquid connector fluidly connects the first bag outlet and the injection device inlet, and the injection device is in an extended position for penetrating an injection site;

- an activation system (100) comprising a trigger (101) which can be moved from a rest position to an activated position, thereby:
- causing the gas supply system to go from its inactive state to its active state, thus causing the second bag to inflate and to compress the first bag;
- and causing the dispensing system to go from its inactive state to its active state, thus allowing the drug contained in the first bag to be delivered to the injection site.

## Description

The invention relates to a drug delivery system.

Particularly, but not exclusively, the invention concerns an on-body drug delivery system, which can be temporarily attached to a patient's body during delivery, thereby allowing long delivery times without requiring someone to hold the system in place.

Such drug delivery systems can include a dispensing system for injecting the drug, said dispensing system being activated by the action of a user on a trigger of the drug delivery system.

This activation can be achieved by means of a mechanical system, which can include an elastic member such as a spring to provide a pushing force for expelling the drug. However, storing the fairly significant amount of energy needed in such elastic members requires space while the drug delivery system needs to remain compact.

Alternatively, this activation can be achieved by means of an electromechanical system. This may require the use of a motor having a fairly significant size to generate sufficient thrust to expel the drug. As a result, drug delivery systems tends to be quite cumbersome and heavy. Besides, the use of electronics components, batteries, etc. is not desirable from an environmental point of view.

Although conventional drug delivery systems are generally satisfactory, room for improvement remains.

The invention concerns a drug delivery system, comprising:
- a first bag configured to contain a drug in liquid form, the first bag having an outlet;
- a second bag without fluid communication with said first bag, the second bag having an inlet, the second bag being arranged adjacent the first bag and configured to be filled with a gas through its inlet so as to inflate and, as a result, to compress the first bag in order to expel the drug from the first bag through the first bag outlet;
- a gas supply system which can be in an inactive state or in an active state, and which comprises:
   - a gas container;
- a gas connector configured to fluidly connect the gas container to the second bag inlet in the active state of the gas supply system, to allow inflating the second bag;
- a dispensing system which can be in an inactive state or in an active state, and which comprises an injection device having an inlet, and a liquid connector configured to fluidly connect the first bag outlet to the injection device inlet, wherein:
   - in the inactive state of the dispensing system, the liquid connector is in a blocking state in which it prevents fluid communication between the first bag outlet and the injection device inlet, and the injection device is in a retracted position, and
   - in the active state of the dispensing system, the liquid connector is in a connecting state in which it fluidly connects the first bag outlet and the injection device inlet, and the injection device is in an extended position in which it can penetrate an injection site;
- an activation system comprising a trigger which can be moved from a rest position to an activated position, thereby:
   - causing the gas supply system to go from its inactive state to its active state, thus causing the second bag to inflate and to compress the first bag;
   - and causing the dispensing system to go from its inactive state to its active state, thus allowing the drug contained in the first bag to flow to the injection device inlet and ultimately to be delivered to the injection site.

Owing to the use of a gas container, which is a device capable of storing a high amount of energy in a small volume, the drug delivery system of the invention can be limited in size and weight as compared to conventional systems. It can therefore be designed as either a wearable system that is intended to remain in place over a long delivery period, or as a handheld system.

Furthermore, no electronics are required to operate the drug delivery system, which has environmental benefits.

Besides, the two-bag arrangement of the invention has several advantages.

On the one hand, this allows the constituent material of a given bag to be adapted to the use of that bag. More specifically, the material of the first bag must be compatible with the drug it contains, while the material of the second bag must be able to withstand the deformation caused by the inflation that second bag. There is no need to select a single material with both the required physical / chemical properties, which can prove difficult.

On the other hand, the arrangement of the invention does not require to manage dynamic tightness, but only static tightness, which is easier to design.

The terms "without fluid communication" mean that no fluid (whether it is a liquid or a gas) can flow from one of the first and second bags towards the other. Besides, a component which "fluidly connects" two elements allows fluid to flow from one of the first and second elements towards the other.

The drug delivery system may further comprise a housing. The housing can have one compartment for receiving the first and second bags, and one compartment for receiving the gas supply system and the dispensing system, the compartments being distinct. Preferably, the housing forms a rigid enclosure for ensuring that the inflation of the second bag entails the compression of the first bag. The first bag and second bag must be able to deform, for being compressed or inflated, respectively. For example, at least part of each bag is made of a flexible material or component.

The first bag can have an inlet port to be filed with the drug, said inlet port preferably being accessible from outside the housing. The gas connector may have a port connected to the second bag inlet.

The injection system may comprise a needle and a catheter. These can both project from a wall of the housing - towards the injection site in use - in the extended position of the injection system.

The transition of the injection device from its retracted position to its extended position, or the transition of the dispensing system from its inactive state to its active state, can be automatic, i.e. automatically caused by the trigger movement. Alternatively, this transition can be manual, i.e. can require a separate action of a user.

The activation system may comprise a gas actuator configured to be moved from a rest position to an operational position by the trigger when the trigger is moved from its rest position to its activated position, so that the gas actuator places the gas supply system in its active state.

The gas actuator is moved by the trigger, for example is pushed by it. Preferably the gas actuator is directly moved by the trigger, i.e. without intermediate part. In other words, with this embodiment, the trigger is in contact with the gas actuator.

The gas actuator may comprise holding means for holding said gas actuator in the rest position and/or holding means for holding said gas actuator in the operational position.

The holding means can prevent movement from one position towards the other, but also from said one position in the opposite direction. The holding means for holding said gas actuator in the rest position and the holding means for holding said gas actuator in the operational position can be the same part or parts or can have at least one common part.

The holding means can comprise at least one protrusion configured to cooperate with an abutment which is a stationary part in the concerned position and regarding the concerned movement to be prevented. Such stationary part can be a housing of the drug delivery system or a part secured to said housing.

The activation system may comprise a liquid actuator configured to be moved from a rest position to an operational position as a result of the trigger moving from its rest position to its activated position, so that the activation system places the dispensing system in its active state.

In an embodiment, the liquid actuator is configured to be moved from its rest position to its operational position by the trigger when the trigger is moved from its rest position to its activated position.

Then, the liquid actuator is moved by the trigger, for example is pushed by it. Preferably the liquid actuator is directly moved by the trigger, i.e. without intermediate part. In other words, with this embodiment, the trigger is in contact with the liquid actuator. In this embodiment, the trigger can activate two different parts, namely the gas actuator and the liquid actuator. In practice, the trigger may have two different points of contact with the mechanism, namely one point of contact with the gas actuator of the gas supply system, and one distinct point of contact with the liquid actuator of the dispensing system.

In another embodiment, the liquid actuator is configured to be moved from its rest position to its operational position by an intermediate member which goes to an active state as a result of the movement of the trigger. In practice, the trigger may have one single point of contact with the mechanism. Said intermediate member may be a spring released by the movement of the gas actuator. In this embodiment, preferably, the liquid actuator is not in contact or direct contact with the trigger.

The liquid actuator may be configured to cause:
- a flow shutter of the liquid connector to move from a closed to an open position, to allow fluid communication between the first bag outlet and the injection device inlet;
- and/or a retaining device of the injection device to be released, to allow the injection device to go from its retracted position to its extended position;
when the liquid actuator is moved from its rest position to its operational position.

The liquid actuator may comprise holding means for holding said liquid actuator in the operational position.

The holding means prevent movement towards the rest position, to ensure that the injection is fully completed. The holding means can comprise at least one protrusion configured to cooperate with an abutment which is a stationary part in the operational position Such stationary part can be a housing of the drug delivery system or a part secured to said housing.

In an embodiment, the trigger can pivot about an axis - with respect to the bags or to the housing when it is present - from its rest position towards its activated position.

The gas actuator and/or the liquid actuator can move by translation from the rest position to the operational position.

The drug delivery system may comprise holding means for holding the trigger in its rest position, to prevent its unwanted movement towards the activated position and/or to prevent its movement in the opposite direction.

Preventing the unwanted movement of the trigger towards the activated position allows preventing untimely transition of the gas supply system and the dispensing system to the active state. Preferably, the holding means can be overcome by the application of a sufficient force on the trigger.

In an embodiment, the gas connector comprises a port fluidly connected to the second bag inlet and a hollow pin which is in fluid communication with said port. Furthermore, the gas container is closed and located at a distance from said hollow pin in the inactive state of the gas supply system, said gas container being movable towards the gas connector up to the active state of the gas supply system in which a wall of the gas container is pierced by said hollow pin to allow gas to flow towards the second bag inlet.

The drug delivery system may comprise holding means for preventing the gas container to untimely move towards the hollow pin when the gas supply system is in the inactive state. These holding means can be overcome with a sufficient effort on the trigger.

The drug delivery system may further comprise holding means for preventing the gas container to move away from the gas connector in the inactive state of the gas supply system. In combination with the holding means preventing a movement towards the hollow pin, they allow avoiding shaking of the gas container in the inactive state.

The drug delivery system may further comprise holding means for holding the gas container in the active state of the gas supply system, preferably both towards and away from the gas connector. The holding means in the inactive state and in the active state can be the same part or parts or can have at least one common part.

The holding means can comprise at least one abutment which is a stationary part in the concerned position and regarding the concerned movement to be prevented. Such stationary part can be a housing of the drug delivery system or a part secured to said housing, including the gas connector.

The holding means can comprise at least one elastically deformable member preferably secured to the housing and capable of cooperating with a part of the gas container for retaining the gas container in the inactive state of the gas supply system, such as at least one outwardly deflectable arm.

The gas connector may comprise a port fluidly connected to the second bag inlet and a hole distinct from the port and in fluid communication with the port, said hole being configured to provide a controlled gas leak. Such a hole is small enough to have little effect on the time it takes to inflate the second bag. However, once the drug delivery system has been used, the hole allows the second bag to slowly deflate, thus allowing the pressure to slowly decrease, and avoiding the risk of explosion. For example, the hole is dimensioned so that the time required to reach a substantially zero pressure in the second bag is at least ten times the time required to inject the whole amount of drug.

In an embodiment, the first bag comprises a first sheet and a second sheet that are superimposed and secured to one another at their peripheral edges, and the second bag comprises a third sheet that is superimposed to the second sheet opposite the first sheet, the second sheet and the third sheet being secured to one another at their peripheral edges.

In other words, this forms a pouch defining two distinct bags having a common middle sheet.

This set of first and second bags can be housed in a rigid compartment of the housing.

Alternatively, first sheet and the third sheet can be rigid. By "rigid" is meant that these sheets are able on their own to withstand the pressure inside the second bag when it is inflated, even if the first and second bags are not enclosed in a rigid compartment. Furthermore, the rigidity of these sheets must not be a hindrance to the inflation of the second bag, i.e. to the drug discharge.

The appropriate rigidity can be obtained by the choice of the constituent material of the sheet or by the thickness of the sheet.

In another embodiment, the first bag and the second bag are initially distinct and are superimposed and secured to one another, for example by gluing or heat sealing.

The first bag may have a cross-sectional shape that diverges towards its outlet. This arrangement avoids the formation of fluid pockets within the first bag, and ensures that substantially all of the drug contained in the first bag is expelled. This prevents underdosing.

Further advantages and advantageous features of the invention are disclosed in the following description and in the dependent claims.

A possible embodiment of the invention will now be described by way of non-limiting examples with reference to the appended figures:
Figures 1 and 2 are perspective views from two different orientations of a drug delivery system according to an embodiment of the invention, with a trigger in a rest position;
Figure 3 is a top view of the drug delivery system, without cover, showing a first and a second bags, a gas supply system, a dispensing system and an activation system, with the trigger in a rest position;
Figure 4 is a partial perspective view of the first and a second bags, gas supply system, dispensing system and activation system, with the trigger in a rest position;
Figure 5 shows a detail of Figure 4;
Figure 6 is a view similar to Figure 4, with the trigger in the activated position,
Figure 7 shows a detail of Figure 6, around the dispensing system, and from another orientation;
Figure 8 is a schematic function diagram of a drug delivery system according to the invention;
Figure 9 schematically shows the set formed by the first and second bags, according to one embodiment;
Figure 10 schematically shows the set formed by the first and second bags, according to another embodiment;
Figures 11a and 11b schematically show an embodiment of a set including the first bag containing a drug and the second bag substantially empty, the set being received in a compartment, in two different cross-sections;
Figures 12a and 12b are views similar to Figures 11a and 11b, respectively, the inflation process of the second bag being in an early stage;
Figures 13a and 13b are views similar to Figures 12a and 12b, respectively, the inflation process of the second bag being in a subsequent stage;
Figures 14a, 14b and 14c are schematically plane views of the first bag, according to several embodiments;
Figures 15a, 15b and 15c are cross-section views of another embodiment of a set including the first bag containing a drug and the second bag substantially empty, respectively when the second bag is substantially empty, during inflation of the second bag, and when the first bag is substantially empty;
Figure 16 is a cinematic diagram of a drug delivery system according to another embodiment of the invention, with a trigger in a rest position;
Figure 17 is a view similar to Figure 16 when the trigger is in the activated position.

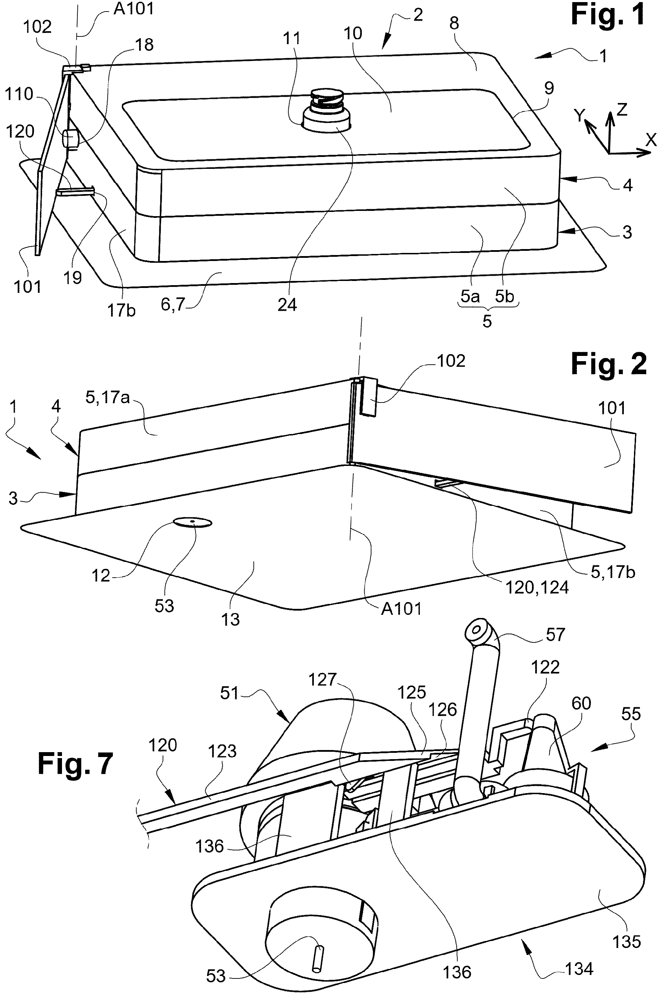

Figures 1 and 2 show an embodiment of a drug delivery system 1 according to the invention.

In this non-limiting example, the drug delivery system 1 comprises a housing 2 which can be rigid and which can have a substantially parallelepiped shape. The housing 2 may comprise a base 3 and a cover 4.

The base 3 can have a peripheral wall 5a and a bottom wall 6 which may comprise a rim 7 extending outwardly from the peripheral wall 5a. The cover 4 can have a peripheral wall 5b and a top wall 8 in which can be formed a window 9 closed by a transparent wall 10 to allow viewing the drug inside of the housing 2. The top wall 8 or the transparent wall 10 may comprise an opening 11 the function of which will be explained later.

In the mounted position of the housing 2, the peripheral wall 5a of the base 3 and the peripheral wall 5b of the cover 4 together form the peripheral wall 5 of the housing 2. Preferably, the peripheral wall 5b of the cover 4 forms the extension of the peripheral wall 5a of the base 3, such that the peripheral wall 5 of the housing 2 comprises four flat portions.

The bottom wall 6 of the base 3 comprises an opening 12 the function of which will be explained later. The face of the bottom wall 6 that is opposite the peripheral wall 5a may be at least partially covered with an adhesive 13, so that the drug delivery system 1 can be temporarily attached to the skin of a patient, at an injection site.

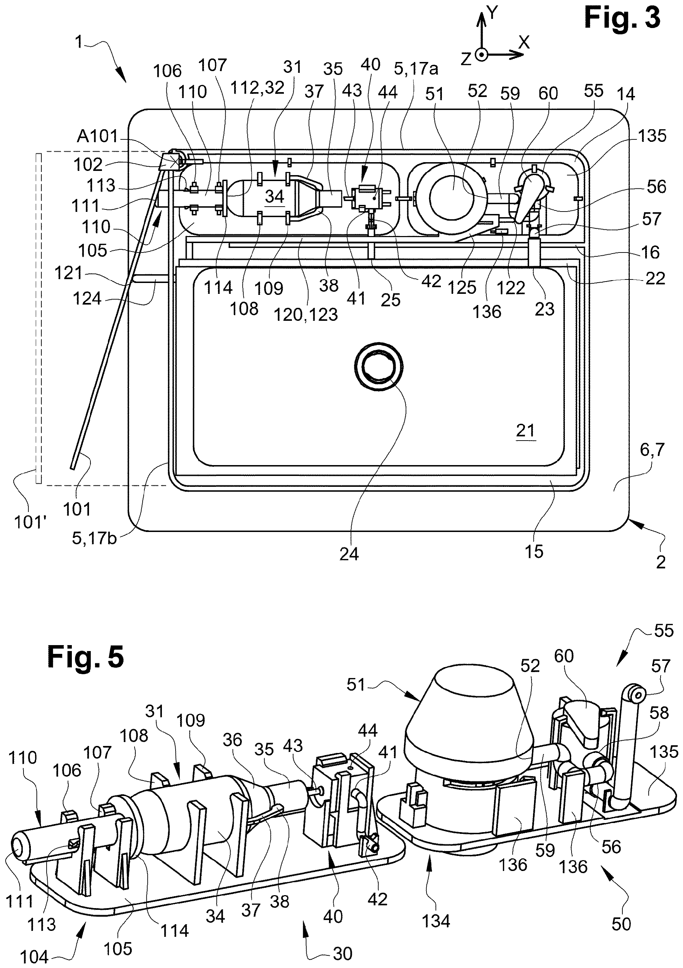

As can be seen in Figure 3, the housing 2 may define a first compartment 14 and a second compartment 15 which may be separated by a partition 16 arranged on the base 3 and/or on the cover 4. The partition 16 is preferably substantially orthogonal to the bottom wall 6 and parallel to one flat portion 17a of the peripheral wall 5 of the housing 2. Each compartment 14, 15 may extend from the bottom wall 6 to the top wall 8 and from two opposite flat portions of the peripheral wall 5 of the housing 2. The first compartment 14 may be less wide than the second compartment 15, for example substantially half the width of the second compartment 15. In the illustrated embodiment, the first compartment 14 is located between the flat portion 17a of the housing peripheral wall 5 and the partition 16.

Direction X is defined as being parallel to the bottom wall 6 are to the partition 16 and flat portion 17a. It corresponds to the main direction of the first compartment 14. Direction Y is defined as being parallel to the bottom wall 6 and orthogonal to direction X. Direction Z is defined as being orthogonal to directions X and Y, i.e. orthogonal to the bottom wall 6.

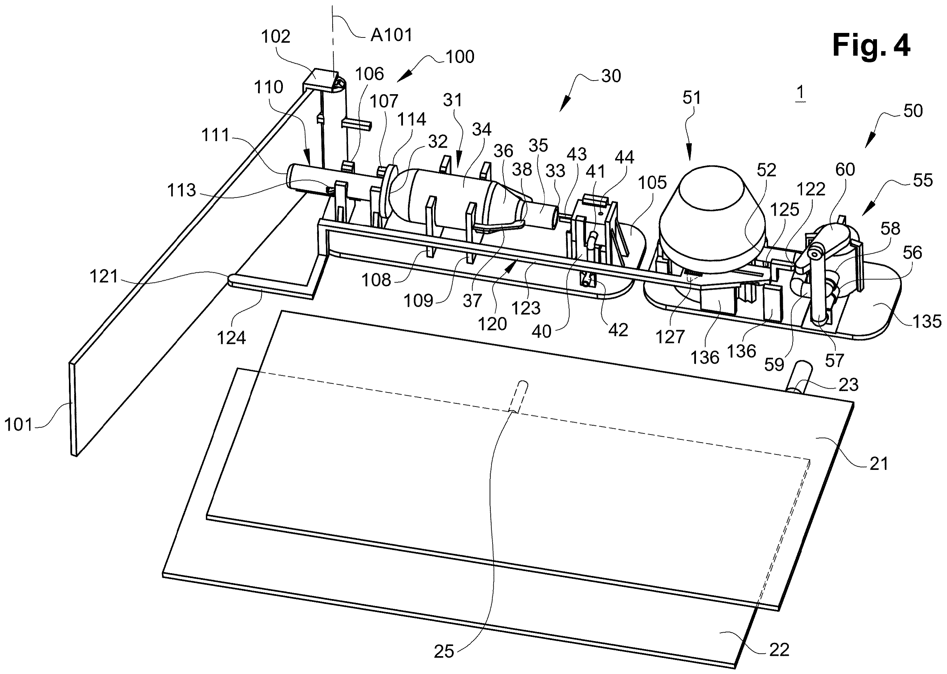

The drug delivery system 1 comprises a first bag 21 which is configured to contain a drug in liquid form. The first bag 21 has an outlet 23 for allowing the drug to be delivered to the injection site. The first bag 21 may also have an inlet port 24 (not shown in figure 4). The inlet port 24 may extend through the opening 11 of the cover 4, so that it is accessible from outside the housing 2.

The drug delivery system 1 also comprises a second bag 22 which has an inlet 25.

There is no possible fluid communication between the first bag 21 and the second bag 22. The second bag 22 is arranged adjacent the first bag 21 and is configured to be filled with a gas through its inlet 25 so as to inflate and, as a result, to compress the first bag 21 in order to expel the drug from the first bag 21 through the first bag outlet 23.

Each of the first and second bags 21, 22 may comprise two sheets of appropriate material that are superimposed and secured to one another at their peripheral edges. The first and second bags 21, 22 may have substantially identical dimensions, when empty.

The first and second bags 21, 22 may be arranged one on top of the other - along direction Z - and received in the second compartment 15 of the housing 2, as shown in figure 3. Preferably, the first bag 21 is located closer to the top wall 8.

The outlet 23 of the first bag 21 and the inlet 25 of the second bag 22 are preferably arranged at edges of the bags 21, 22 which are located close to the partition 16, therefore substantially facing the first compartment 14.

The drug delivery system 1 further comprises a gas supply system 30 for inflating the second bag 22, and a dispensing system 50 for delivering the drug contained in the first bag 21 to the injection site. Both the gas supply system 30 and the dispensing system 50 are caused to go from an inactive state to an active state by means of an activation system 100 which comprises a trigger 101, when said trigger 101 is moved from a rest position to an activated position.

In the illustrated embodiment, both the gas supply system 30 and the dispensing system 50 are received in the first compartment 14, the dispensing system 50 being located further from the trigger 101 than the gas supply system 30.

An embodiment of the activation system 100 will now be described, with reference to figures 3, 4 and 5, in which the activation system 100 is in the rest position.

The trigger 101 is located outside the housing 2. It may extend along one flat portion 17b of the peripheral wall 5 of the housing 2 that is adjacent the flat portion 17a bordering the first compartment 14. The flat portion 17b is thus parallel to directions X and Y.

With respect to direction X, the term "forward" is used for a direction extending from the trigger 101 towards the inside of the housing 2, while the term "backward" is used for the opposite direction, i.e. a direction extending from the inside of the housing 2 towards the trigger 101.

The trigger 101 may be plate-shaped. The trigger 101 can pivot with respect to the housing 2 about an axis A 101, which is for example substantially coincident with the edge between the flat portions 17a, 17b of the housing peripheral wall 5, i.e. which substantially extends along direction Z. The trigger 101 can pivot:
- from its rest position, in which the trigger 101 makes an angle α with the flat portion 17b;
- towards its activated position, in which the trigger 101 makes a lower angle with the flat portion 17b, i.e. has moved closer to the flat portion 17b.

However, other types of movement of the trigger from its rest position to its activated position can be envisaged, such as a translation.

There may be provided a stop member 102 for preventing the trigger 101 to move from its rest position in the direction opposite the flat portion 17b, i.e. backward. The stop member 102 can be secured to the housing 2.

The activation system 100 may further comprise a gas actuator 110 which is configured to be moved from a rest position to an operational position by the trigger 101 when the trigger 101 is moved from its rest position to its activated position. The gas actuator 110 is intended to cooperate with the gas supply system 30.

In the exemplary embodiment, the gas actuator 110 is inserted in a hole 18 of the flat portion 17b and extends both outside and inside the housing 2, transversely with respect to the trigger 101. For example, the gas actuator 110 extends along direction X. The gas actuator 110 has a first end 111, located outside the housing 2, which is in contact or very close to the trigger 101, and a second end 112, located inside the housing 2. The gas actuator 110 can take the shape of a rod.

The gas actuator 110 may be mounted on a support 104 secured to the housing 2. The support 104 may comprise a plate 105 secured to the bottom wall 6, parallel to the bottom wall 6, and located in the first compartment 14, and at least one leg extending from the plate 105 opposite the bottom wall 6, substantially along direction Z. For example, the support 104 comprises a set of first legs 106 which face each other along direction Y and a set of second legs 107 which face each other along direction Y and which are located further away from the trigger 101 than the first legs 106. The gas actuator 110 may be supported between the first legs 106 and between the second legs 107.

The movement of the gas actuator 110 from its rest position to its operational position may be a translation along direction X, forward, as the trigger 101 pushes the first end 111 of the gas actuator 110 during its move towards its activated position.

The gas actuator 110 can comprise a protrusion 113, or a set of protrusions 113 facing each other along direction Y, which, in the rest position of the gas actuator 110, are in abutment against the first legs 106 on the side of the first end 111. Thus, the protrusions 113 prevent the gas actuator 110 from moving forward towards its operational position. As a result, as the first end 111 of the gas actuator 110 is in contact with the trigger 101, the protrusions 113 prevent the trigger 101 from moving towards its activated position or, more precisely, prevent this movement from occurring unintentionally. In an embodiment, the protrusions 113 may be arranged on a tongue capable of deflecting inwardly above a predetermined forward force exerted on the gas actuator 110.

Besides, the gas actuator 110 can comprise a plate 114 forming a flange extending outwardly at the second end 112. The plate 114 can be disc-shaped. In the rest position of the gas actuator 110, the plate 114 is in abutment against the second legs 107, on the side opposite the first legs 106. Thus, the plate 114 prevents the gas actuator 110 from moving backward from its rest position.

Therefore, the protrusions 113 and plate 114 together form holding means for holding the gas actuator 110 in its rest position.

Furthermore, the protrusions 113 abutting against the first legs 106, and the stop member 102, together form holding means for holding the trigger 101 in its rest position, to prevent its unwanted movement towards the activated position and in the opposite direction, respectively.

The activation system 100 may further comprise a liquid actuator 120 which is configured to be moved from a rest position to an operational position by the trigger 101 as a result of the trigger 101 moving from its rest position to its activated position. The liquid actuator 120 is intended to cooperate with the dispensing system 50.

In the exemplary embodiment, the liquid actuator 120 is inserted in a hole 19 of the flat portion 17b and extends both outside and inside the housing 2, transversely with respect to the trigger 101. For example, the liquid actuator 120 generally extends along direction X. The liquid actuator 120 has a first end 121, located outside the housing 2, which is in contact or very close to the trigger 101, and a second end 122, located inside the housing 2.

The liquid actuator 120 can take the shape of a bar, for example an angled bar.

The liquid actuator 120 can comprise a main portion 123 which is for example substantially straight and located in the first compartment 14. This main portion 123 may run along the supply system 30 and the dispensing system 50, between the partition 16 and the supply system 30 / dispensing system 50. As, in the illustrated embodiment, the dispensing system 50 is located further from the trigger 101 than the gas supply system 30, the main portion 123 extends beyond the second end 112 of gas actuator 110 in the forward direction.

The liquid actuator 120 can also comprise an input portion 124 extending backward from the main portion 123 up to the first end 121. A part of this input portion 124 which extends along direction X may be offset from the main portion 123 in direction Y, opposite axis A101. The liquid actuator 120 can also comprise an output portion 125 extending forward from the main portion 123 up to the second end 122. A part of this output portion 125 which extends along direction X may be offset from the main portion 123 in direction Y, opposite the partition 16, i.e. towards the dispensing system 50, in order to interact with it, as will be explained later.

The liquid actuator 120 may be mounted on a support 134 secured to the housing 2. The support 134 may comprise a plate 135 secured to the bottom wall 6, parallel to the bottom wall 6, and located in the first compartment 14, as well as two stands 136. The output portion 125 is supported by the stands 136. For example, each stand 136 is plate-shaped and extends in a plane parallel to directions X and Z. The stands 136 have a common median plane, have the same height from the plate 135 and are located spaced apart the one from the other in direction X.

The movement of the liquid actuator 120 from its rest position to its operational position may be a translation along direction X, forward, as the trigger 101 pushes the first end 121 of the liquid actuator 120 during its move towards its activated position. With a liquid actuator 120 in the form of an angled bar designed such that the first end 121 of the liquid actuator 120 is offset opposite axis A101, the distance between said first end 121 and said axis A101 is increased. As a result, the pivoting movement of the trigger 101 about axis A101 entails a relatively large translational movement of the liquid actuator 120 along direction X.

As can be seen in figure 7, in an embodiment, the output portion 125 comprises a groove 126 which extends parallel to direction X and which is open towards the plate 135. The stands 136 of the support 134 are received in the groove 126 and thus guide the translational movement or the liquid actuator 120 from its rest position to its operational position.

The output portion 125 may further comprise a tooth 127 which is located in the groove 126 and extends towards the plate 135. The tooth 127 may be part of a flexible tongue formed in the groove 126. In the rest position of the liquid actuator 120, the tooth 127 is located backward with respect to the stands 136, as shown in figure 4. The abutment of the tooth 127 against one stand 136 may prevent the liquid actuator 120 from moving up to its operational position or, more precisely, prevent this movement from occurring unintentionally.

The gas supply system 30 will now be described with reference to figures 3 to 5, in which the gas supply system 30 is in its inactive state from which it can go to its active state.

The gas supply system 30 comprises a gas container 31, which can be in the form of a gas cartridge. The gas container 31 has a bottom 32 which is located in contact with of very close to the plate 114 of the gas actuator 110, opposite the trigger 101, and an end wall 33 which is opposite the bottom 32. Initially, the end wall 33 is closed so that no gas can flow out of the gas container 31. Said end wall 33 can form a septum.

The gas supply system 30 further comprises a gas connector 40 which is configured to fluidly connect the gas container 31 to the second bag inlet 25 in the active state of the gas supply system 30, to allow inflating the second bag 22. The gas connector 40 is fixed with respect to the housing 2. It can be secured to the plate 105 of the support 104 on which the gas actuator 110 is movably mounted.

The gas connector 40 comprises a port 41 which is fluidly connected to the second bag inlet 25, for example by means of a pipe 42. The gas connector 40 also comprises a hollow pin 43 which is in fluid communication with said port 41.

In practice, the gas connector 40 can form a box, for example cube-shaped, having faces orthogonal to directions X, Y and Z. The port 41 may be arranged in a face orthogonal to direction Y that faces the partition 16, the gas connector 40 and second bag 22 being preferably arranged so that the inlet 25 and the port 41 - or the outlet of pipe 42 - substantially face each other. Besides, the hollow pin 43 extends along direction X, backward, i.e. towards the gas container 31, the hollow pin 43 and the gas container end wall 33 substantially facing each other. The fluid communication between the port 41 and the hollow pin 43 may be provided by inner fluid ducts in the gas connector 40.

The hollow pin 43 is capable of piercing the gas container end wall 33. But in the inactive state of the gas supply system, the gas container 31 is located at a distance from the hollow pin 43, so that the gas container 31 can remain closed.

The drug delivery system 1 preferably comprises holding means for preventing the gas container 31 to untimely move towards the hollow pin 43 when the gas supply system 30 is in the inactive state.

In practice, the gas container 31 may comprise a main portion 34 having a cylindrical shape and a neck 35 which extends forward form the main portion 34 and has a smaller diameter, the main portion 34 and neck 35 being connected by means of a conical portion 36 which tapers forward.

For example, the support 104 comprises a set of third legs 108 which face each other along direction Y and a set of fourth legs 109 which face each other along direction Y and which are located further away from the trigger 101 than the third legs 108. The main portion 34 of the gas container 31 may be supported between the third legs 108 and between the fourth legs 109.

In an embodiment, the holding means for preventing the forward movement of the gas container 31 when the gas supply system 30 is in the inactive state comprise two arms 37. Each arm 37 extends from one of the fourth legs 109 along direction X and has a free end provided with a bulge 38 protruding towards the gas container 31. The arms 37 are arranged in a facing relationship along direction Y, on both sides of the gas container 31. The arms 37 are arranged at the conical portion 36 of the gas container 31 and elastically biased towards each other so as to tighten the gas container 31 together. Furthermore, the bulges 38 are in abutment against a shoulder formed between the conical portion 36 and the neck 35 of the gas container 31.

Thus, in the inactive state, the gas container 31 is held between the plate 114 and the bulges 38 of the arms 37, thereby being prevented from shaking with respect to the housing 2.

The dispensing system 50 will now be described with reference to figures 3 to 5, in which the dispensing system 50 is in its inactive state from which it can go to its active state.

The dispensing system 50 may be secured to the plate 135 of the support 134.

The dispensing system 50 comprises an injection device 51 that can be arranged next to the output portion 125 of the liquid actuator 120, for example along direction Y. The injection device 51 has an inlet 52 for receiving the drug to be injected, and an outlet 53 for delivering the drug. A shown in figures 2 and 7, the outlet 53 opens towards the outside of the housing 2, preferably through the bottom wall 6 of the base 3.

The injection device 51 may be manual or automatic. It can be similar to the injection device described in US10737038, US10195342, US10434247 or US11458248.

The dispensing system 50 also comprises a liquid connector 55 configured to fluidly connect the first bag outlet 23 to the injection device inlet 52. In practice, the liquid connector 55 may have an inlet port 56 which is fluidly connected to the first bag outlet 23, for example by means of a pipe 57, and an outlet port 58 which is fluidly connected to the injection device inlet 52, for example by means of a pipe 59.

The inlet port 56 may be arranged so as to face the partition 16 along direction Y, the liquid connector 55 and first bag 21 being preferably arranged so that the outlet 23 and the inlet port 56 - or the outlet of pipe 57 - substantially face each other. The fluid communication between the inlet port 56 and the outlet port 58 may be provided by inner fluid ducts in the liquid connector 55.

The liquid connector 55 may further comprise a lever 60 which cooperates with the liquid actuator 120, for example by being in contact or very closed to the second end 122 of the liquid actuator 120.

In the illustrated embodiment, the liquid connector 55 is located forward with respect to the injection device 51, in the first compartment 14. The liquid connector 55 may comprise a valve, such as a rotating valve. However, other embodiments can be envisaged.

In the inactive state of the dispensing system 50, the liquid connector 55 is in a blocking state in which it prevents fluid communication between the first bag outlet 23 and the injection device inlet 52. For example, the liquid connector 55 may comprise a flow shutter (not shown) which is then in a closed position. Furthermore, the injection device 51 is in a retracted position in which no injection is possible into an injection site. For example, with an injection device 51 comprising a needle or a needle and a catheter, these do not project beyond the bottom wall 6 of the housing base 3. The injection device 51 may be provided with a retaining device which maintains said injection device 51 in the retracted position. Part of the retaining device may comprise the output portion 125 of the liquid actuator 120, which may be partially located in the casing of the injection device 51 and thus prevent the movement of inner parts of the injection device 51.

From the above described state of the drug delivery system 1, in which the trigger 101 is in its rest position, and the gas supply system 30 and dispensing system 50 in their inactive state, a user can operate the drug delivery system 1 in order to perform injection to at the injection site.

To that end, the trigger 101 is moved from its rest position to its activated position, here by a user pressing the trigger 101 in order to make it pivot about axis A101. The pressure should preferably by applied at a distance from axis A101 to require less effort. In a variant, as schematically illustrated in figure 3, there may be provided an additional outer part 101' to be pushed according to direction X with respect to the housing 2 and which causes the trigger 101 to pivot about axis A101. For example, the outer part 101' can belong to a casing which is arranged around the trigger 101 and which is mounted on the housing 2 so that it can move in translation relative to said housing 2. Such an arrangement may be more convenient or less confusing for some users, as it can simplify the understanding of the drug delivery system.

On the one hand, pivoting the trigger 101 results in the liquid actuator 120 being pushed forward in direction X, up to its operational position. This causes the dispensing system 50 to go from its inactive state to its active state in which the liquid connector 55 is in a connecting state and the injection device 51 is in an extended position, as explained below according to a possible embodiment.

The second end 122 of the liquid actuator 120 is moved forward, which causes the lever 60 to rotate and thus the flow shutter of the liquid connector 55 to move from its closed position to an open position, thereby allowing a fluid communication between the first bag outlet 23 and the injection device inlet 52. The drug which is forced to exit the first bag 21 as said first bag 21 is compressed by the second bag 22 thus flows up to the injection device inlet 52.

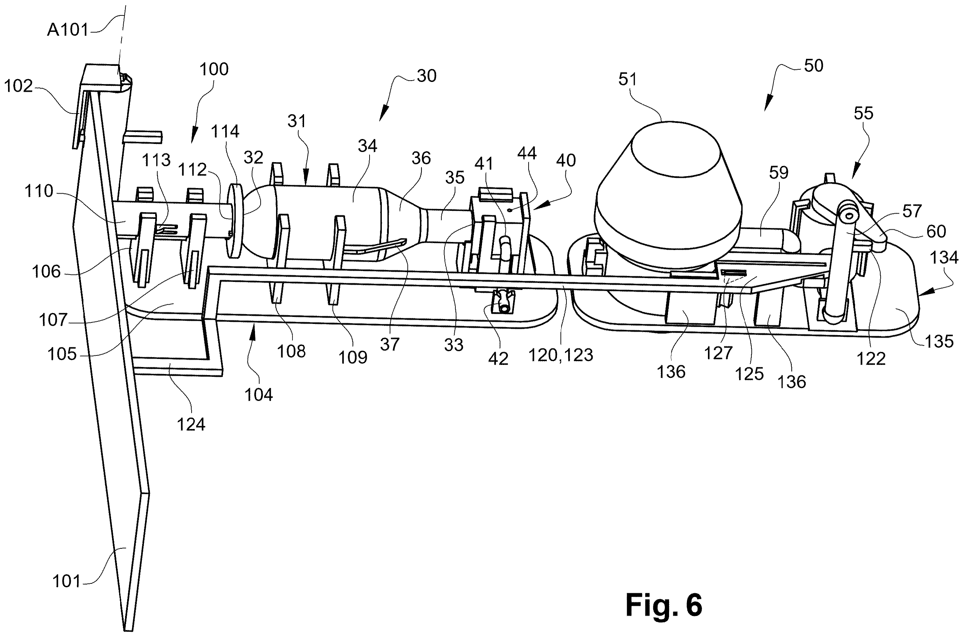

In addition, the output portion 125 of the liquid actuator 120 is also moved forward, with a force sufficient to make the tooth 127 go beyond the first stand 136 it contacts, until it reaches the gap between the two stands 136. Then, the output portion 125 of the liquid actuator 120 no longer interacts with the injection device 51 as it is preferably located fully outside the casing of the injection device 51, as can be seen in figure 6. As a result the retaining device of the injection device 51 is released, which allows the injection device 51 to go from its retracted position to its extended position. In this extended position, the needle and/or catheter project beyond the housing 2, so as to penetrate the injection site, as schematically shown in figure 7. Thus, the drug contained in the first bag 21, which has reached the injection device inlet 52 as the liquid connector 55 is in its open position, can flow up to injection device outlet 53 and can be delivered to the injection site.

On the other hand, pivoting the trigger 101 results in the gas actuator 110 being pushed forward in direction X. Above a predetermined pushing force allowing the protrusions 113 to move past the first legs 106, the gas actuator 110 moves forward, thereby pushing the gas container 31. The forward movement of the gas container 31 causes the arms 37 to deflect outwardly or otherwise be released, thereby freeing the gas container 31 which can then move towards the gas connector 40. The hollow pin 43 can thus pierce the end wall 33 of the gas container 31. As a result, the second bag inlet 25 is fluidly connected to the gas container 31 through the gas connector 40. Thus, the gas contained in the gas container 31 flows towards the second bag 22 and inflates it. Consequently, the second bag 22 compresses the first bag 21.

The gas supply system 30 is then in its active state and the gas actuator 110 is in its operational position.

Thus, the trigger 101 activates both the dispensing system 50 and the gas supply system 30. The drug delivery system 1 may be designed so that the events listed below occur in the following order: pricking of the patient, opening of the liquid connector 55, piercing of the gas container 31. This specific order can result from an appropriate dimensioning of the different parts, in particular by providing a slight offset in part travels.

The drug delivery system is then in the state illustrated in figures 6 and 7.

In this state, the protrusions 113 abut against the first legs 106, on the side of the second legs 107, thereby preventing the gas actuator 110 from moving backward. Furthermore, the gas container 31 is pushed forward by the plate 114 of the gas actuator 110 to be in abutment against the gas connector 40. Thus, the gas actuator 110 is held in the operational position, and the gas container 31 is held in its forward position.

Besides, the tooth 127 of the liquid actuator 120 abut against the first stand 136, on the side of the second stand 136, thereby preventing the liquid actuator 120 from moving backward. Furthermore, the second end 122 of the liquid actuator 120 can be blocked forward by the lever 60 of the liquid connector 55. Thus, the liquid actuator 120 is held in the operational position.

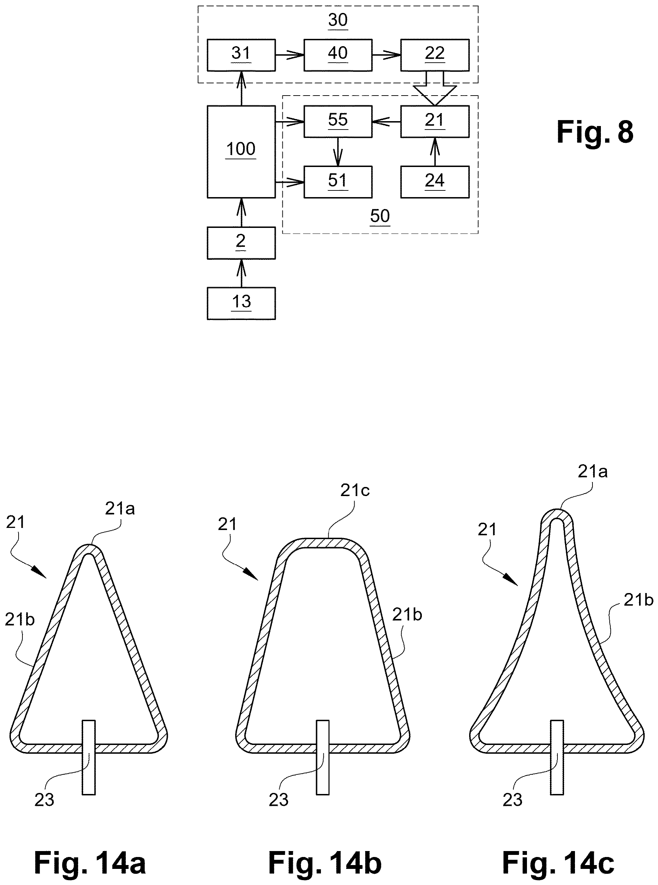

Figure 8 provides a schematic function diagram of the drug delivery system 1. It shows that the activation system 100, which is mounted on the housing 2 itself attached to the patient by means of the adhesive 13, acts on both the dispensing system 50 and the gas supply system 30.

More specifically, on the one hand, the activation system 100 causes the injection device 51 to move to its extended position, thus penetrating the an injection site, and the liquid connector 55 to move to its connecting state, thus allowing communication with the injection device 51.

On the other hand, the activation system 100 causes the gas container 31 to be pierced, thus allowing gas to flow through the gas connector 40 to the second bag 22. As a result, the second bag 22 inflates and compresses the first bag 21, providing a drug flow towards the liquid connector 55 and ultimately to the injection site through the injection device 51.

Figure 8 also shows the inlet port 24 allowing the first bag 21 to be filled with a drug.

The gas connector 40 may comprise a hole 44 distinct from the port 41 and in fluid communication with the port 41. The hole 44 is configured to provide a controlled gas leak so as to allow the second bag 22 to slowly deflate. This avoids the risk that too much pressure remains in the second bag 22 after injection is complete, which could entail an explosion.

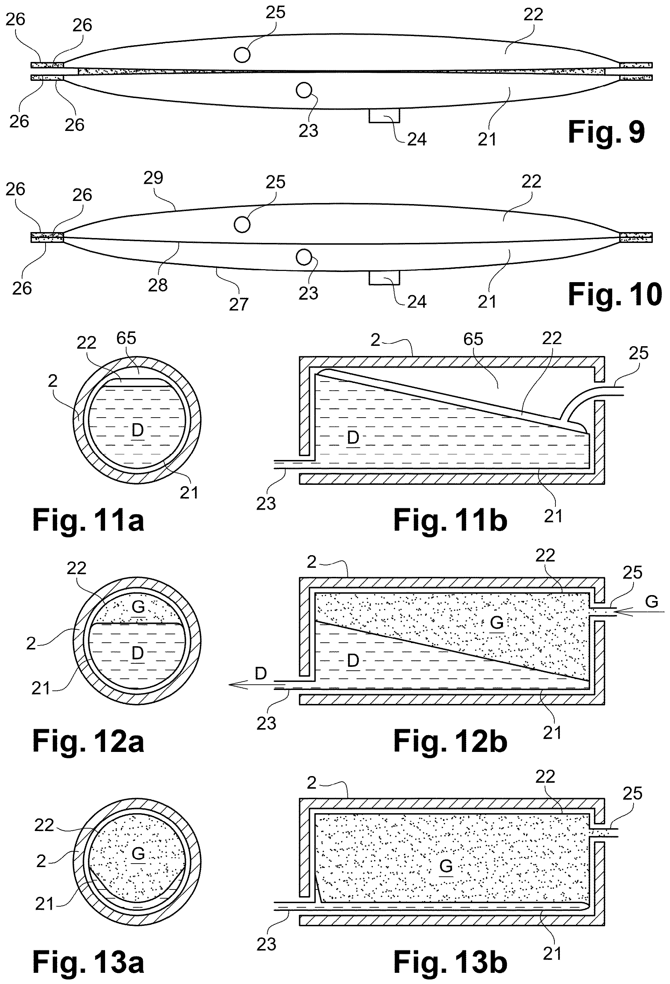

Two different embodiments of the first and second bags 21, 22 are shown in figures 9 and 10.

In the embodiment of figure 9, the first bag 21 and the second bag 22 are initially distinct and are superimposed and secured to one another, for example by gluing or heat sealing. Each bag 21, 22 can typically be made of two sheets, for example two sheets of plastic material, that are superimposed and secured to one another at their peripheral edges 26.

In the embodiment of figure 10 the first bag 21 comprises a first sheet 27 and a second sheet 28 that are superimposed and secured to one another at their peripheral edges 26. Furthermore, the second bag 22 comprises a third sheet 29 that is superimposed to the second sheet 28 opposite the first sheet 27, the second sheet 28 and the third sheet 29 being secured to one another at their peripheral edges 26. In other words, the second sheet 28 is common to the first bag 21 and the second bag 22, and the two bags together form a pouch.

Figures 10a to 12b show the progressive filling of the second bag 22 with gas G and the resulting compression of the first bag 21, leading to the drug D being discharged through the outlet 23.

The first and second bags 21, 22 are received in a rigid enclosure, here the second compartment 15 of the housing 2.

Initially, as shown in figures 11a and 11b, there is an empty space 65 in the housing 2, adjacent the second bag 22, to allow the second bag 22 to inflate. When gas enters the second bag 22, the second bag 22 first occupies the empty space 65 and then, as pressure increases, it compresses the first bag 21. When injection is complete, as shown in figures 13a and 13b, the first bag 21 is empty and the second bag 22 entirely fills the inner space of the second compartment 15.

As can be seen in figures 11b and 12b, the first bag 21 may have a cross-sectional shape that diverges towards its outlet 23. With this arrangement, the inflated second bag 22 first compresses the thinnest part of the first bag 21, i.e. the part located furthest from the outlet 23. Consequently, this avoids the formation of fluid pockets within the first bag, and ensures that substantially all of the drug contained in the first bag is expelled.

As shown in figures 14a to 14c, the first bag 21 may have a cross-sectional shape that diverges towards its outlet 23, in plane view.

In the embodiment of figure 14a, the first bag 21 has a substantially triangular shape, with straight edges 21b converging at an apex 21a opposite the outlet 23. In the embodiment of figure 14b, the first bag 21 also have straight edges 21b that converge opposite the outlet 23; however the part opposite the outlet 23 is an edge 21c which is not reduced to an apex or a point. In the embodiment of figure 14c, the first bag 21 has curved edges 21b converging at an apex 21a opposite the outlet 23, the curved edges 21b preferably being concave.

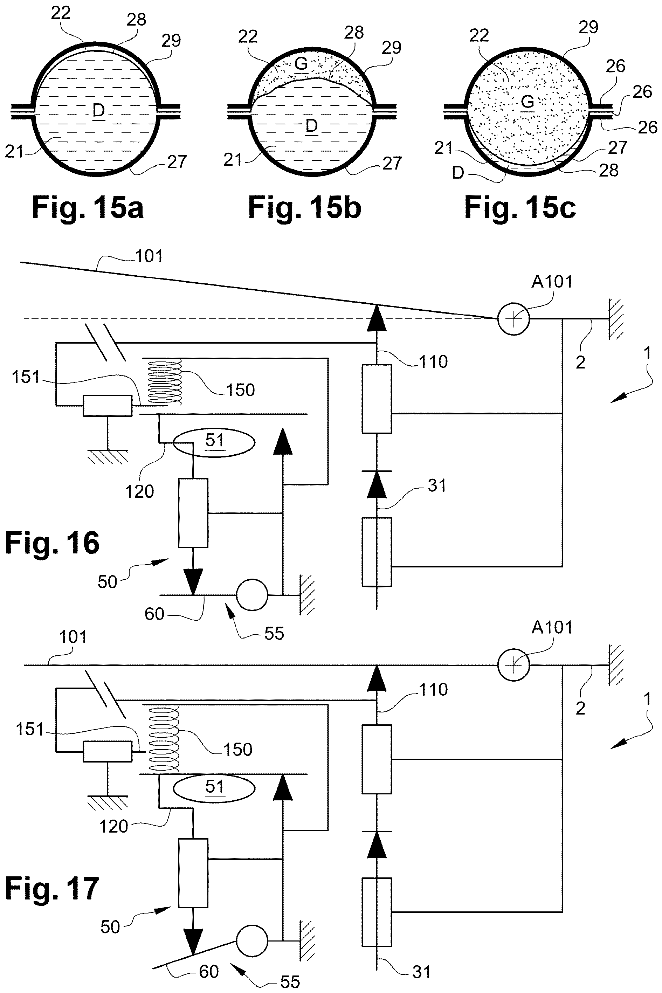

Reference is now made to figures 15a to 15c which show a variant of the invention, when the first and second bags 21, 22 are according to the embodiment of figure 10, i.e. with a common second sheet 28. It has to be noted that the circular cross-section of the housing 2 in figures 15a, 15b, 15c is not limiting and is only for illustration purposes.

It may be envisaged to design the first sheet 27 and the third sheet 29, i.e. the outer sheets of the pouch formed by the two bags 21, 22, as rigid. With this arrangement, no housing is needed. Indeed, the first sheet 27 and the third sheet 29 both form a rigid wall which allows controlling the inflation of the second bag 22 and forcing it to compress the first bag 21.

Initially, as shown in figure 15a, the first bag 21 is filled with drug D and the second bag 22 is empty. Then, gas G is progressively introduced in second bag 22 which thus inflates and compresses the first bag 21, the drug D flowing towards the dispensing system 50. In figure 15c, the injection is nearly complete: the first bag 21 is nearly empty and the second bag 22 nearly entirely fills the initial global volume.

In the previously disclosed embodiment, the liquid actuator 120 is moved from its rest position to its operational position by the trigger 101. Indeed, when moving from its rest position to its activated position, the trigger 101 pushes the liquid actuator 120 towards its operational position.

However, other embodiments can be envisaged, such as the one schematically illustrated in figures 16 and 17.

In this embodiment, the liquid actuator 120 is moved from its rest position to its operational position by an intermediate member which goes to an active state as a result of the movement of the trigger 101.

More precisely, the liquid actuator 120 is not directly actuated by the trigger 101. Instead, the drug delivery system 1 comprises an intermediate member capable of acting on the liquid actuator 120. This intermediate member can be a spring 150 which is in an inactive state in the rest position of the activation system 100, as shown in figure 16. For example, in this inactive state, the spring 150 is compressed, and held in this position by a retainer 151.

The movement of the trigger 101 to its activated position causes the gas actuator 110 to move to its operational position, as previously described, and further causes the release of the spring 150. For example, the gas actuator 110 causes the retainer 151 to be retracted, thereby freeing the spring 150.

As a result, the spring 150 urges the liquid actuator 120 towards its operational position. Consequently, the dispensing system 50 goes to its active state, in which the liquid connector 55 is in its connecting state and the injection device 51 is in its extended position, so that injection of the drug can be performed.

With this configuration, there is provided a single point of contact between the trigger 101 and the rest of the system. Preferably, the drug delivery system 1 can be designed with this point of contact located failry close to axis A101. Thus, the pushing force on the gas actuator 110 is quite high, which is required for opening the gas container 31 by means of the hollow pin 43. The displacement of the gas actuator 110 is small, but sufficient to ensure the opening of the the gas container 31. A larger displacement may be required for a correct insertion of the injection device 51 in the injection sit,. Such a larger displacement is achieved thanks to the spring 150, even with a small displacement of the gas actuator 110.

The invention is not limited to the embodiments described above by way of examples but it rather comprises all the technical equivalents and variants of the means described as well as their combinations.

It is to be understood that the present invention is not limited to the embodiments described above and illustrated in the drawings; rather, the skilled person will recognize that many changes and modifications may be made within the scope of the appended claims.

## Claims

1. A drug delivery system (1), comprising:
- a first bag (21) configured to contain a drug in liquid form, the first bag (21) having an outlet (23);
- a second bag (22) without fluid communication with said first bag (21), the second bag (22) having an inlet (25), the second bag (22) being arranged adjacent the first bag (21) and configured to be filled with a gas through its inlet (25) so as to inflate and, as a result, to compress the first bag (21) in order to expel the drug from the first bag (21) through the first bag outlet (23);
- a gas supply system (30) which can be in an inactive state or in an active state, and which comprises:
- a gas container (31);
- a gas connector (40) configured to fluidly connect the gas container (31) to the second bag inlet (25) in the active state of the gas supply system (30), to allow inflating the second bag (22);
- a dispensing system (50) which can be in an inactive state or in an active state, and which comprises an injection device (51) having an inlet (52), and a liquid connector (55) configured to fluidly connect the first bag outlet (23) to the injection device inlet (52), wherein:
- in the inactive state of the dispensing system (50), the liquid connector (55) is in a blocking state in which it prevents fluid communication between the first bag outlet (23) and the injection device inlet (52), and the injection device (51) is in a retracted position, and
- in the active state of the dispensing system (50), the liquid connector (55) is in a connecting state in which it fluidly connects the first bag outlet (23) and the injection device inlet (52), and the injection device (51) is in an extended position in which it can penetrate an injection site;
- an activation system (100) comprising a trigger (101) which can be moved from a rest position to an activated position, thereby:
- causing the gas supply system (30) to go from its inactive state to its active state, thus causing the second bag (22) to inflate and to compress the first bag (21);
- and causing the dispensing system (50) to go from its inactive state to its active state, thus allowing the drug contained in the first bag (21) to flow to the injection device inlet (52) and ultimately to be delivered to the injection site.

2. The drug delivery system according to claim 1, **characterized in that** the activation system (100) comprises a gas actuator (110) configured to be moved from a rest position to an operational position by the trigger (101) when the trigger (101) is moved from its rest position to its activated position, so that the gas actuator (110) places the gas supply system (30) in its active state.

3. The drug delivery system according to claim 2, **characterized in that** the gas actuator (110) comprises holding means (113, 114) for holding said gas actuator (110) in the rest position and/or holding means (113, 114) for holding said gas actuator (110) in the operational position.

4. The drug delivery system according to any one of claims 1 to 3, **characterized in that** the activation system (100) comprises a liquid actuator (120) configured to be moved from a rest position to an operational position as a result of the trigger (101) moving from its rest position to its activated position, so that the activation system (100) places the dispensing system (50) in its active state.

5. The drug delivery system according to claim 4, **characterized in that** the liquid actuator (120) is configured to be moved from its rest position to its operational position:
- by the trigger (101) when the trigger (101) is moved from its rest position to its activated position;
- or by an intermediate member (150) which goes to an active state as a result of the movement of the trigger (101).

6. The drug delivery system according to claim 4 or 5, **characterized in that** the liquid actuator (120) is configured to cause:
- a flow shutter of the liquid connector (55) to move from a closed to an open position, to allow fluid communication between the first bag outlet (23) and the injection device inlet (52);
- and/or a retaining device of the injection device (51) to be released, to allow the injection device (51) to go from its retracted position to its extended position;
when the liquid actuator (120) is moved from its rest position to its operational position.

7. The drug delivery system according to any one of claims 4 to 6, **characterized in that** the liquid actuator (120) comprises holding means (127) for holding said liquid actuator (120) in the operational position.

8. The drug delivery system according to any one of claims 1 to 7, **characterized in that** it comprises holding means (102, 113, 106) for holding the trigger (101) in its rest position, to prevent its unwanted movement towards the activated position and/or to prevent its movement in the opposite direction.

9. The drug delivery system according to any one of claims 1 to 8, **characterized in that** the gas connector (40) comprises a port (41) fluidly connected to the second bag inlet (25) and a hollow pin (43) which is in fluid communication with said port (41), and **in that** the gas container (31) is closed and located at a distance from said hollow pin (43) in the inactive state of the gas supply system (30), said gas container (31) being movable towards the gas connector (40) up to the active state of the gas supply system (30) in which a wall (33) of the gas container (31) is pierced by said hollow pin (43) to allow gas to flow towards the second bag inlet (25).

10. The drug delivery system according to claim 9, **characterized in that** it comprises holding means(37, 38) for preventing the gas container (31) to untimely move towards the hollow pin (43) when the gas supply system (30) is in the inactive state.

11. The drug delivery system according to any one of claims 1 to 10, **characterized in that** the gas connector (40) comprises a port (41) fluidly connected to the second bag inlet (25) and a hole (44) distinct from the port (41) and in fluid communication with the port (41), said hole (44) being configured to provide a controlled gas leak.

12. The drug delivery system according to any one of claims 1 to 11, **characterized in that** the first bag (21) comprises a first sheet (27) and a second sheet (28) that are superimposed and secured to one another at their peripheral edges (26), and **in that** the second bag (22) comprises a third sheet (29) that is superimposed to the second sheet (28) opposite the first sheet (27), the second sheet (28) and the third sheet (29) being secured to one another at their peripheral edges (26).

13. The drug delivery system according to claim 12, **characterized in that** the first sheet (27) and the third sheet (29) are rigid.

14. The drug delivery system according to any one of claims 1 to 11, **characterized in that** the first bag (21) and the second bag (22) are initially distinct and are superimposed and secured to one another, for example by gluing or heat sealing.

15. The drug delivery system according to any one of claims 1 to 14, **characterized in that** the first bag (21) has a cross-sectional shape that diverges towards its outlet (23).
